# EUROPEAN PATENT APPLICATION

(11) **EP 1 992 691 A1**
(43) Date of publication of application: **19.11.2008**
(21) Application number: 07009862.9
(22) Date of filing: 16.05.2007
(51) Int. Cl.: C12N 15/09, C12N 5/10, C07K 14/01, C12Q 1/68, G01N 33/53

(54) **New TTV sequences for diagnosis, prevention and treatment of childhood leukaemia**

(71) Applicant: Deutsches Krebsforschungszentrum, Stiftung des öffentlichen Rechts, 69120 Heidelberg (DE)
(72) Inventor: The designation of the inventor has not yet been filed
(74) Representative: Schüssler, Andrea

(57) **Abstract**

Described are (a) a particular rearranged TTV polynucleic acid and (b) probes and primers comprising part of said TTV polynucleic acid. Moreover, polypeptides encoded by said TTV polynucleic acid as well as antibodies binding to said polypeptide are described. Finally, various uses of said compounds for diagnosis of future development of childhood leukaemia and for other malignant proliferations (cancer) and autoimmune diseases and a vaccine for prevention or treatment of said disease are described.

## Description

The present invention relates to a particular rearranged TTV polynucleic acid and probes and primers comprising part of said TTV polynucleic acid. The present invention also relates to a polypeptide encoded by said TTV polynucleic acid as well as antibodies binding to said polypepeptide. Finally, the present invention relates to the use of said compounds for diagnosis of future development of childhood leukaemia and for other malignant proliferations (cancer) and autoimmune diseases and a vaccine for prevention or treatment of said diseases.

Several attempts have been made to link the ubiquitous Torque teno (TT) viruses to the aetiology of a specific disease. These viruses were originally isolated from a patient suffering from post transfusion hepatitis, but subsequent studies were unable to demonstrate a direct role for TT virus in the pathogenesis of hepatitis or hepatocellular carcinoma.

TT viruses are circular single-stranded DNA viruses belonging to the Anelloviruses which range in size from 2 kb to 3,8 kb. They have been isolated from humans and primates, as well as from pigs (2878 bp), dogs (2797 bp) and cats (2064 bp) (1, 2, 3, 4). Their genomes consist of 2 main open reading frames (ORF) and additional smaller ORFs resulting from putative splicing events (1, 5). The putative non-coding region has a very high GC content and about 180 bp in this region are highly conserved among these viruses (6, 7). Their genome organization is similar to that of chicken anemia virus (CAV), which is vertically transmitted through the egg from infected parent chickens, causing anemia through lymphoid depletion in the progeny.

Peripheral blood mononuclear cells act as reservoir for TT viruses, whereas viral transcription and replication occur in bone marrow cells. Investigations into a possible association between the presence of TT virus infections and leukemia or lymphoma have resulted in contradictory results (8, 9, 10).

Thus, the technical problem underlying the present invention is to provide means (or markers) for diagnosis of leukemia or diagnosis of a disposition to said diseases.

The solution to said technical problem is achieved by providing the embodiments characterized in the claims. Previously, the full-length genomes of a spectrum of TT virus types from the spleen of a Hodgkin's disease patient were isolated (1). One group of 6 isolated complete genomes revealed the reorganization of the largest open reading frame, ORF1. Despite less than 1% overall nucleotide variation between these isolates, single nucleotide differences resulted in the formation of two or several smaller ORFs within this region of the genome.

In the present experiments it was elucidated whether these rearranged TT virus genomes may be of significance in the pathogenesis of disease. *In vitro* replicated DNA and virus particle formation after transfection of the full-length tth8 genome into a Hodgkin's lymphoma-derived cell line could be demonstrated. The presence of additional off-size viral DNA revealed intragenomic rearrangement events occurring in the viral genome. Analyses of the transcription patterns of tth8 and the ORF1-interrupted tth7 (1) in a number of lymphoma and T-cell leukaemia cell lines indicated differential additional splicing events and intragenomic rearrangement leading to open reading frames which could not be deducted from the genomic sequence. In addition, the presence of rearranged TT virus genomes ranging from 172 bp to full-length genomes *in vivo* in sera taken from pregnant mothers whose children later developed childhood leukaemia could be demonstrated. Although a certain region of the TT virus genome seemed to be retained in these rearranged isolates, existing ORFs were sometimes completely deleted, but mostly modified to induce new putative proteins. Additional new ORFs were also introduced. A possible in vivo selection for specific rearranged molecules was evidenced by the presence of one isolate (561 bp) in 11 serum samples suggesting this TT virus isolate may contribute to the initiation of disease.

### Brief description of the drawings

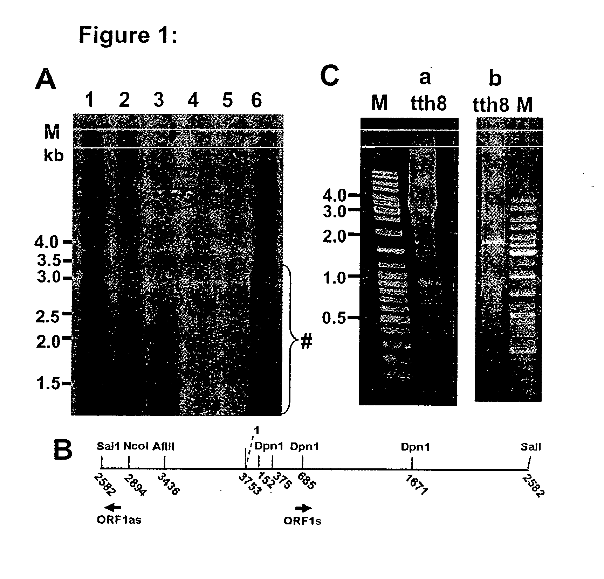

Figure 1: *In vitro* replication and intragenomic rearrangement of tth8 in the L428 cell line
   **(A)** Southern blot analyses of total cellular DNA harvested at different time periods after transfection. DNA was digested with DpnI to fragmentize input viral DNA (#). Hybridization was performed with labelled tth8 DNA. Replication of genomic tth8 DNA (SalI linearized, 3.7kb) increased from day 2 to day 7 following transfection. Lane 1 - 1 day, lane 2 - 2 days, lane 3 - 7 days, lane 5 - negative control of untransfected cells, lane 6 - positive control for DpnI digestion of untransfected cellular DNA and linearized tth8 genome. M - DNA size marker.
   **(B)** Schematic presentation of the tth8 genome after linearization with SalI and indicating the DpnI digestion sites. ORF1as and ORF1s primers were used to amplify almost full-length tth8 DNA indicating the recircularization of the linearized tth8 genome used for transfection (data not shown).
   **(C)** (a) PCR amplification of full-length tth8 DNA (3.7kb), as well as shorter rearranged subviral fragments 2 days after transfection in L428 cells. Primers A and B were used for amplification. (b) Control long PCR amplification of re-ligated cloned genomic tth8 DNA mixed with L428 cellular DNA. M - DNA size marker.

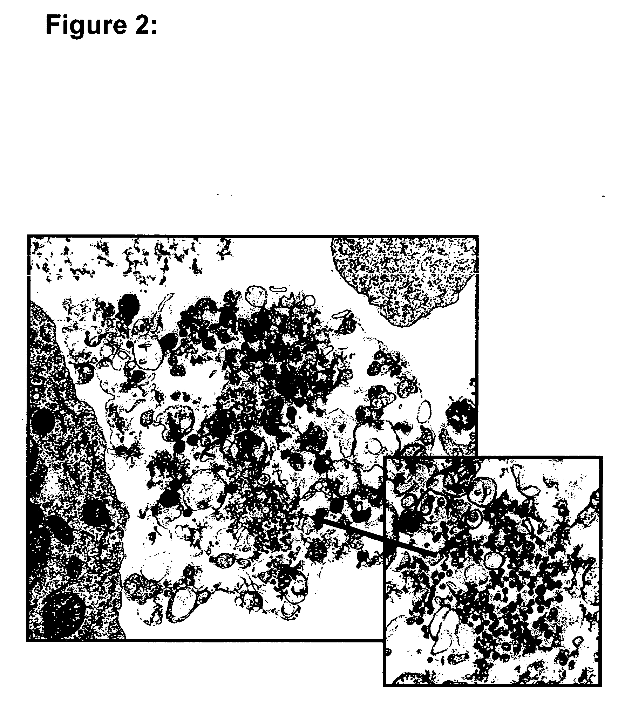

Figure 2: Electronmicrograph of L428 cells 4 days after transfection with linearized full-length tth8 DNA
   TT virus-like particles within a destructed and probably apoptotic cell are visible, measuring approximately 30-40nm in diameter.
Figure 3:
   **(a)** Schematic presentation of tth7 and tth8 transcripts
      Full lines indicate RNA sequences and dotted lines introns or deleted regions. Nucleotide positions of the junction sites are indicated. Putative ORFs are presented by boxes and the predicted protein size of each indicated. Primers used for amplification are shown by arrows.
      **A-** ORFs as predicted from the full-length transcript of TT virus genomic DNA.
      **B-** mRNA species (a to e) obtained after 5'RACE amplification using the 5'NGSP primer. Transcripts a and a1, as well as b, b1 and e, were identical to each in length other respectively, but sequence analyses resulted in putative proteins varying in size.
      **C-** mRNA transcripts (f to h) identified by RT-PCR amplification of the ORF1 using h7g47s and h7g47as primers.
      **D-** Transcripts i to n identified by RT-PCR using primers ORF1s and ORF1as.
   **(b)** The cell lines L428, BJAB and HSB2 were transfected with tth8 DNA, RNA isolated 2 days after transfection and oligo(dT) cDNA synthesis perfomed. Regions within the ORF1 were amplified
      **A-** RT-PCR amplification using primers ORF1s and ORF1as. Lanes 1, 9 and 13 - negative controls, lane 2 - tth8 genomic DNA as positive control for amplification, lanes 3-5, 6-8 and 10-12 - 30, 33 and 36 cycles of RT-PCR amplification for L428, BJAB and HSB2 samples, respectively. Amplicons indicated the presence of transcripts a and b in L428 and BJAB, whereas no transcripts were detected in HSB2 cells.
      **B-** RT-PCR amplification using primers h7g43f and h7g43r (supplementary data). Amplicons indicated the presence of a non-spliced transcript, as well as the additional transcripts g and h. Lanes 3-5 - 30, 33 and 36 cycles of RT-PCR amplification respectively, lane 1 - negative control, lane 2 - positive control of tth8 genomic DNA amplification, M - DNA size marker.

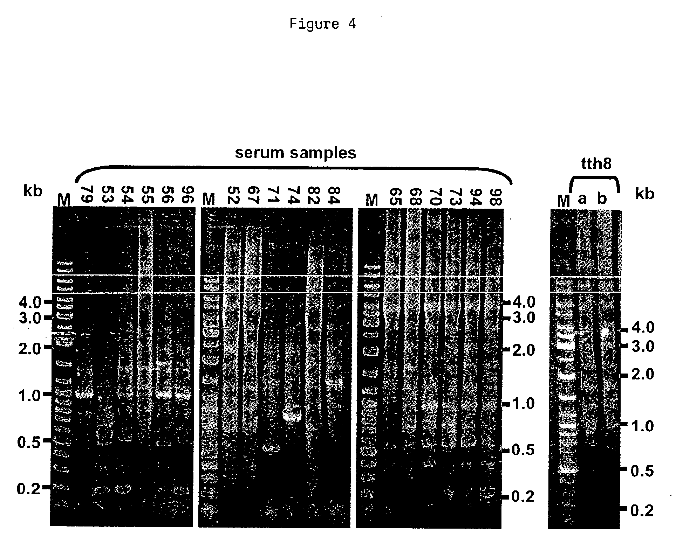

Figure 4: Examples of the long PCR amplification of DNA from individual serum samples using jt34f1s/jt34f2as and tth25-1s/tth25-2as
   Full length TT virus genomes (3.5 to 4 kb) were amplified as well as off-size rearranged subviral fragments. Serum sample numbers are indicated at the top. Long PCR amplification was controlled by amplification of re-ligated and rolling circle amplified tth8 genomic DNA mixed with placenta DNA (lanes a = 10² copies and b 10³ copies of the tth8 genome). M - DNA size marker.

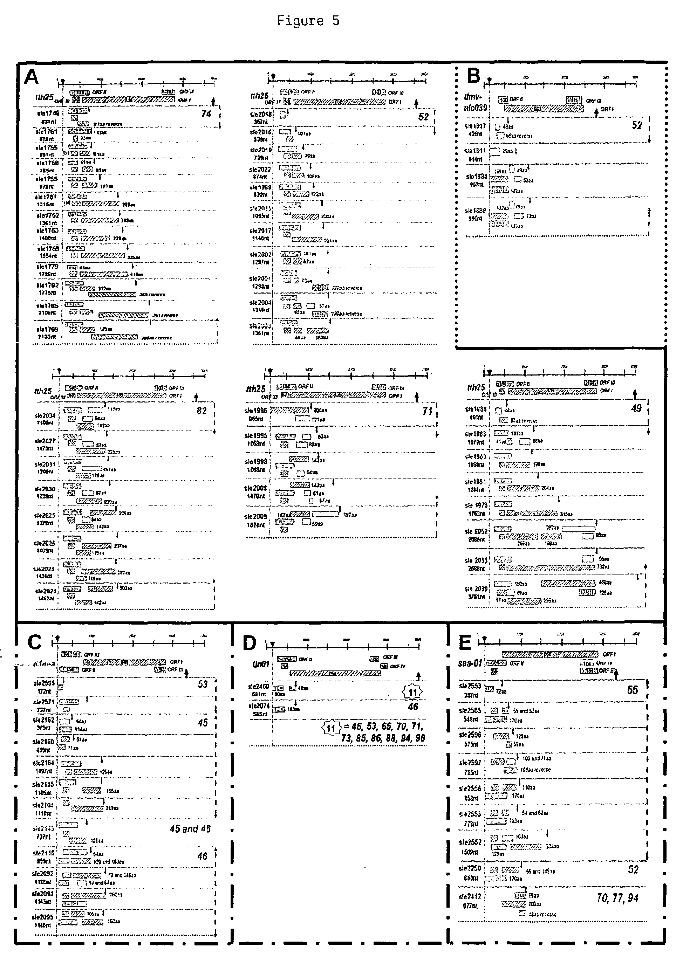

Figure 5: Examples of the genomic organization of the rearranged subviral isolates from individual serum samples (numbers indicated on right side of each block)
   The genomic organization of the closest related known TT virus is indicated at the top of each series - A - group closest related to tth25 (acc. no. aj620222), B - to tlmv-nlc030 (acc. no. ab0038631), C - to tchn-a (acc. no. af345526), D - to tjn01 (acc. no. ab028668) and E - to saa-01 (acc. no. ab060597). ORFs are indicated by filled boxes if homologous to known TT virus ORFs and open boxes if no homology was identified. Size deviation from known TT virus ORFs are indicated. Accession numbers of subviral isolates are presented as supplementary data.
Figure 6: Breakpoint positions (arrows) in the intragenomic rearranged subviral isolates in relation to the tth25 genome
   Boxes indicate the positions of the respective TT virus ORFs.
Figure 7: Nucleotide sequence of the TTV isolate

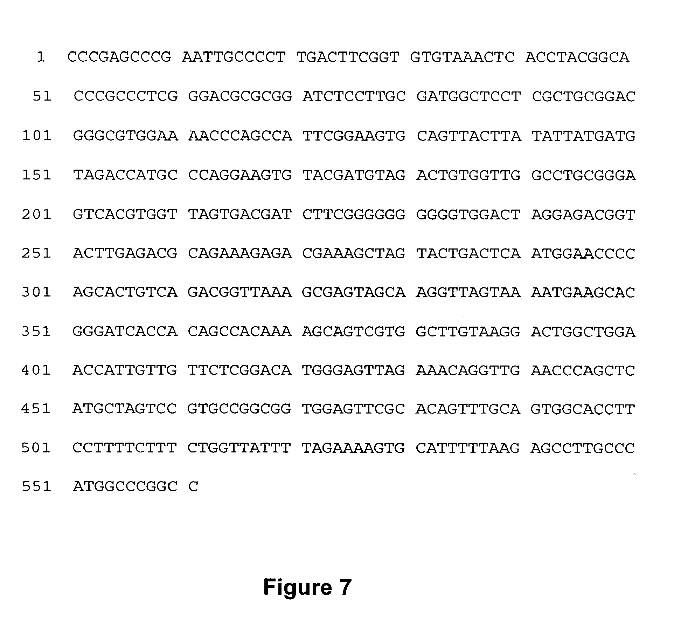

Accordingly, the present invention relates to a TTV polynucleic acid comprising: (a) the nucleotide sequence depicted in Figure 7; (b) a nucleotide sequence having at least 90% identity to the nucleotide sequence of (a), or (c) a fragment of the nucleotide sequence of (a) or (b).

The term "polynucleic acid" refers to a single-stranded or double-stranded nucleic acid sequence. A polynucleic acid may consist of deoxyribonucleotides or ribonucleotides, nucleotide analogues or modified nucleotides or may have been adapted for diagnostic or therapeutic purposes. A polynucleic acid may also comprise a double stranded cDNA clone which can be used, for example, for cloning purposes.

The TTV polynucleic acid of the invention can be prepared according to well-known routine methods, for example, by (a) isolating the entire DNA from a sample, (b) detecting the TTV sequence by hybridization or PCR and (c) cloning of the TTV sequence into a vector. The TTV polynucleic acid of the invention or fragments thereof can be integrated into the host genome or can be present extrachromosomally.

Also included within the present invention are sequence variants of the polynucleic acid of the invention containing either deletions and/or insertions of one or more nucleotides, especially insertions or deletions of one or more codons, mainly at the extremities of oligonucleotides (either 3' or 5') and which show at least 90%, 95%, 98% or 99% identity to said polynucleic acid sequence of the invention. Polynucleic acid sequences according to the present invention which are similar to the sequence as shown in Figure 7 can be characterized and isolated according to any of the techniques known in the art, such as amplification by means of sequence-specific primers, hybridization with sequence-specific probes under more or less stringent conditions, sequence determination of the genetic information of TTV etc.

Furthermore, the present invention relates to an oligonucleotide primer comprising part of the TTV polynucleic acid of the present invention, said primer being capable of acting as primer for specifically sequencing or specifically amplifying the nucleic acid of a certain TTV isolate containing a nucleotide sequence of the invention.

The term "primer" refers to a single stranded DNA oligonucleotide sequence capable of acting as a point of initiation for synthesis of a primer extension product which is complementary to the nucleic acid strand to be copied. The length and the sequence of the primer must be such that they allow to specifically prime the synthesis of the extension products. Preferably the primer is at least about 10, preferably at least 15 nucleotides. Specific length and sequence will depend on the complexity of the required DNA or RNA targets, as well as on the conditions of primer use such as temperature, ionic strength etc. The amplification primers do not have to match exactly with the corresponding template sequence to warrant proper amplification. The amplification method used can be either polymerase chain reaction (PCR), ligase chain reaction (LCR), nucleic acid sequence-based amplification (NASBA), transcription-based amplification system (TAS), strand displacement amplification (SDA) or amplification by means of Qβ replicase or any other suitable method to amplify nucleic acid molecules using primer extension. During amplification, the amplified products can be conveniently labelled either using labelled primers or by incorporating labelled nucleotides. Labels may be isotopic (³²P, ³⁵S, etc.) or non-isotopic (biotin, digoxigenin, etc.).

The present invention also relates to an oligonucleotide probe comprising part of the TTV polynucleic acid of the present invention, said probe being capable of acting as a hybridization probe for specific detection of the nucleic acid of a certain TTV isolate containing a nucleotide sequence of the present invention.

The term "probe" refers to single stranded sequence-specific oligonucleotides which have a sequence which is complementary to the target sequence of the TTV polynucleic acid to be detected. Preferably, these probes are about 5 to 50 nucleotides long, more preferably from about 10 to 25 nucleotides. The probe can be fixed to a solid support, i.e., any substrate to which an oligonucleotide probe can be coupled, provided that it retains its hybridization characteristics and provided that the background level of hybridization remains low. Usually the solid substrate will be a microtiter plate, a membrane (e.g. nylon or nitrocellulose) or a microsphere (bead). Prior to application to the membrane or fixation it may be convenient to modify the nucleic acid probe in order to facilitate fixation or improve the hybridization efficiency. Such modifications may encompass homopolymer tailing, coupling with different reactive groups such as aliphatic groups, NH_{z} groups, SH groups, carboxylic groups, or coupling with biotin or haptens.

The present invention also relates to a vector containing a TTV polynucleic acid, oligonucleotide primer or oligonucleotide probe of the invention allowing, e.g., expression, mutagenesis or a modification of a sequence by recombination of DNA sequences. Preferably, the vectors are plasmids, cosmids, viruses, bacteriophages and other vectors usually used in the field of genetic engineering. Vectors suitable for use in the present invention include, but are not limited to the T7-based expression vector for expression in mammalian cells and baculovirus-derived vectors for expression in insect cells. Preferably, the polynucleic acid of the invention or part thereof is operatively linked to the regulatory elements in the recombinant vector of the invention that guarantee the transcription and synthesis of an mRNA in prokaryotic and/or eukaryotic cells that can be translated. The nucleotide sequence to be transcribed can be operably linked to a promoter like a T7, metallothionein I or polyhedrin promoter.

The present invention also relates to recombinant host cells transiently or stably containing the TTV polynucleic acid (or fragments thereof) or vectors of the invention. A host cell is understood to be an organism that is capable to take up *in vitro* recombinant DNA and, if the case may be, to synthesize the polypeptids encoded by the polynucleotides of the invention. Preferably, these cells are prokaryotic or eukaryotic cells, for example mammalian cells, bacterial cells, insect cells or yeast cells.

A further embodiment of the invention relates to a polypeptide or fragment thereof which is encoded by a TTV polynucleic acid of the invention. Such polypeptide is, e.g., useful per se for diagnosis or as an immunogen for raising antibodies that can be used for diagnostic purposes. The polypeptide has preferably a length of at least eight amino acids, more preferably of at least 15 amino acids and, particularly preferred, of at least 25 amino acids. Such polypeptide can be produced, e.g., by cultivating a host cell of the invention under conditions allowing the synthesis of the polypeptide and the polypeptide is subsequently isolated from the cultivated cells and/or the culture medium. Isolation and purification of the recombinantly produced polypeptide may be carried out by conventional means including preparative chromatography and affinity and immunological separations. These polypeptides, however, not only comprise recombinantly produced polypeptides but include isolated naturally occurring polypeptides, synthetically produced polypeptides, or polypeptides produced by a combination of these methods. Means for preparing such polypeptides or related polypeptides are well understood in the art.

The present invention also relates to an antibody that binds specifically to a polypeptide as defined above. Preferably, said antibody specifically recognizes an epitope that is not present on polypeptides of several other TTV isolates. The term "antibody", preferably, relates to antibodies which consist essentially of pooled monoclonal antibodies with different epitopic specifities, as well as distinct monoclonal antibody preparations. Monoclonal antibodies are made from an antigen containing fragments of the polypeptides of the invention by methods well known to those skilled in the art. As used herein, the term "antibody" (Ab) or "monoclonal antibody" (Mab) is meant to include intact molecules as well as antibody fragments (such as, for example, Fab and F(ab') 2 fragments) which are capable of specifically binding to protein. Fab and f(ab')2 fragments lack the Fc fragment of intact antibody, clear more rapidly from the circulation, and may have less non-specific tissue binding than an intact antibody. Thus, these fragments are preferred, as well as the products of a FAB or other immunoglobulin expression library. Moreover, antibodies of the present invention include chimeric, single chain, and humanized antibodies.

For certain purposes, e.g. diagnostic methods, the TTV polynucleic acid, polypeptide or antibody of the present invention can be detectably labeled, for example, with a radioisotope, a bioluminescent compound, a chemiluminescent compound, a fluorescent compound, a metal chelate, or an enzyme.

The present invention also relates to a diagnostic kit containing a TTV polynucleic acid, an oligonucleotide primer, an oligonucleotide probe, a polypeptide and/or an antibody of the present invention.

For hybridization based assays, according to the hybridization solution (SSC, SSPE, etc.), the probes should be stringently hybridized to the target (with or without prior amplification) at their appropriate temperature in order to attain sufficient specificity. However, by slightly modifying the DNA probes, either by adding or deleting one or a few nucleotides at their extremities (either 3' or 5'), or substituting some non-essential nucleotides (i.e. nucleotides not essential to discriminate between types) by others (including modified nucleotides or inosine) these probes or variants thereof can be caused to hybridize specifically at the same hybridization conditions (i.e. the same temperature and the same hybridization solution). Also changing the amount (concentration) of probe used may be beneficial to obtain more specific hybridization results.

Suitable assay methods for purposes of the present invention to detect hybrids formed between the oligonucleotide probes and a TTV polynucleic acid in a sample may comprise any of the assay formats known in the art, such as the conventional dot-blot format, sandwich hybridization or reverse hybridization. For example, the detection can be accomplished using a dot blot format, the unlabelled amplified sample being bound to a membrane, the membrane being incorporated with at least one labelled probe under suitable hybridization and wash conditions, and the presence of bound probe being monitored. An alternative and preferred method is a "reverse" dot-blot format, in which the amplified sequence contains a label. In this format, the unlabelled oligonucleotide probes are bound to a solid support and exposed to the labelled sample under appropriate stringent hybridization and subsequent washing conditions. It is to be understood that also any other assay method which relies an the formation of a hybrid between the nucleic acids of the sample and the oligonucleotide probes according to the present invention may be used.

Diagnosis can also be carried out by detecting a polypeptide of the invention or an antibody against this polypeptide in a sample. This diagnostic method uses, e.g., an antibody specifically binding to the polypeptide of the invention and allows said diagnosis, e.g., by ELISA and the antibody is bound to a solid support, for example, a polystyrene microtiter dish or nitrocellulose paper, using techniques known in the art. Alternatively, said method is based on a RIA and said antibody is marked with a radioactive isotope. Preferably, the antibody is labeled. Suitable antibody assay labels are known in the art and include enzyme labels, such as, glucose oxidase, and radioisotopes, such as iodine (¹²⁵I, ¹²¹I), carbon (¹⁴C), sulfur (³⁵S), tritium (³H), indium (¹¹²In), and technetium rhodamine, and biotin.

The present invention also relates to the use of a TTV polynucleic acid, an oligonucleotide primer, an oligonucleotide probe, a polypeptide and/or an antibody of the present invention as an early marker for the future development of childhood leukaemia.

Finally, the present invention also relates to a vaccine for immunizing a mammal against an infection by a TTV characterized by a polynucleic acid of the present invention comprising a polypeptide of the invention in a pharmaceutically acceptable carrier. A "vaccine" is an immunogenic composition capable of eliciting protection against TTV characterized by a polynucleic acid of the present invention, whether partial or complete. A vaccine may also be useful for treatment of an individual, in which case it is called a therapeutic vaccine.

Pharmaceutically acceptable carriers include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers and inactive virus particles. Such carriers are well known to those of ordinary skill in the art.

Preferred adjuvants to enhance effectiveness of the vaccine include, but are not limited to aluminim hydroxide (alum), N-acetyl-muramyl-L-threonyl-D-isoglutamine (thr-MDP), N-acetyl-normuramyl-Lralanyl-D-isoglutamine (nor-MDP), N-acetylmuramyl-L-alanyl-D-isoglutaminyl-L-alanine-2-(1'-2'-dipalmitoyl-sn-glycero-3-hydroxy-phosphoryloxy)-ethylamine (MTP-PE) and RIBI, which contains three components extracted from bacteria, monophosphoryl lipid A, trehalose dimycolate, and cell wall skeleton (MPL, TDM and CWS) in a 2% squalene/Tween 80 emulsion. Any of the 3 components MPL, TDM or CWS may also be used alone or combined 2 by 2. Additionally, adjuvants such as Stimulon (Cambridge Bioscience, Worcester, MA) or SAF-1 (Syntex) may be used. Further, Complete Freund's Adjuvant (CFA) and Incomplete Freund's Adjuvant (IFA) may be used for non-human applications and research purposes.

The vaccine typically will contain pharmaceutically acceptable vehicles, such as water, saline, glycerol, ethanol, etc. Additionally, auxiliary substances, such as wetting or emulsifying agents, pH buffering substances, preservatives, and the like, may be included in such vehicles. Typically, the vaccine is prepared as injectable, either as liquid solution or suspension, solid form suitable for solution in, or suspension in, liquid vehicles prior to injection may also be prepared. The vaccine also may be emulsified or encapsulated in liposomes for an enhanced adjuvant effect.

The present invention also relates to the use of a polypeptide of the invention for the preparation of a vaccine for immunization of a mammal against TTV infection said TTV comprising a polynucleic acid of the present invention.

The following examples illustrate the invention.

### Example 1

### Material Methods

### (A) Cell culture and transfection

L428 (Hodgkin lymphoma), Jurkat (human T cell leukemia), HSB-2 (T-cell leukemia), BJAB (Epstein-Barr virus-negative Burkitt's lymphoma), and Raji (EBV-positive Burkitt's lymphoma) cell lines were maintained in RPMI-1640 culture medium supplemented with 10% FCS and 0.01% penicillin/streptomycin in 5% CO₂ at 37°C. HepG2 (human undifferentiated hepatocellular carcinoma) and 293 (human embryo kidney) cell lines were maintained using Dulbecco's minimum essential medium supplemented with 10% FCS and 0.01% streptomycin. Transfections were performed in the Nucleofector™II transfection device (Amaxa Biosystems, Cologne, Germany) using the respective protocol recommended for each cell type by the manufacturers (Amaxa Nucleotransfection Kit V, Amaxa Biosystems). Two µg of DNA were used to transfect 1x10⁶ cells in the case of the 293 cell line and 5x10⁶ for all other cell lines. The plasmid pmaxGFP (Amaxa Biosystems) was used to measure transfection efficiency in each transfection experiment.

### (B) Serum samples

The children of the 550,000 women who gave birth in Finland or Iceland during 1975-1997 formed a joint study cohort that was followed up for cancer in the offspring before age 15 years through national cancer registries. For each index mother-case pair, three or four control mother-child pairs were selected matched for age of mother and time point of serum sampling. First trimester serum samples were retrieved from the Finnish or Icelandic Maternity Cohort Biobanks from mothers of 342 ALL and 61 other leukaemia cases, as well as from 1,216 matched control mothers, for a previous study on viral antibodies in maternal sera and childhood leukaemia risk (11). For the present experiments, a sub sample of 30 Finnish mothers who gave birth to children who were diagnosed with childhood leukaemia between 0-1.6 years of age were selected for study, together with serum samples from matched control mothers of similar age, having been sampled at the same time and where the control children had been followed until the leukaemia diagnosis was made in the case children. The rationale for selecting the samples from mothers to the youngest cases of leukaemia for this exploratory study was to increase the probability that critical carcinogenic events had occurred already in utero.

### (C) DNA isolation and PCR amplification

The full-length TT virus-tth8 (ACC. NO. AJ620231) and -tth7 (ACC. NO. AJ620230) genomes were each cloned and characterized as described previously (1). Full-length genomes were excised from the plasmid DNA, purified and used for transfection assays. L428 cells transfected with the full-length tth8 DNA were harvested two days after transfection and total cellular DNA extracted. In a second approach the cells were centrifuged at 300 g, after which the supernatant was centrifuged at 2750 g. All pellets were resuspended in 0.5% SDS/TE buffer, treated with 10 µg RNase A (30 min, 37°C) and 200 µg Proteinase K (1 h, 50°C) before the DNA was extracted with phenol/chloroform and precipitated with ethanol. The isolated DNA was treated with DpnI (Fermentas, St.Leon-Rot, Germany) to remove the input plasmid DNA used for transfection.

DNA from serum samples was extracted using the High Pure Viral Nucleic Acid Kit (Roche, Basel, Switzerland). DNA was amplified using the TempliPhi rolling circle amplification (TempliPhi Kit, GE Healthcare, Munich, Germany) followed by long PCR amplification using the TaKaRa LA Taq enzyme (TAKARA BIO INC., Otsu, Japan) and primers for the relevant genomic regions of the TT viruses tth25 (primers tth25-1s/tth25-2as, tth25-7s/tth25-8as) (1) and jt34f (primers jt34f-1s/jt34f-2as, jt34f-3s/jt34f-4as (7) (Table 1).

**Table 1**

| **a: Primers used for RT-PCR and RACE** | | |
|---|---|---|
| **Primer** | **Nucleotide positions in tth8** | **SEQUENCE 5' - 3'** |
| ORF1 s* | 598-618, *EcoRI* | TAT T*GA ATT C*AT GGC ATG GGG ATG GTG GAA |
| ORF1 as** | 2827-2801, *NotI* | GCA GGC GGC CGC GAG CCG TAG GCA TGG GTT TAC ATG GAC CCC |
| h7g43s | 568-588 | CCT CAC AGG TTG ATT CGA GAC |
| h7g43as | 1404-1384 | ACC TGG AAG CTG ATG CAA GTG |
| 5'GSPas | 2660-2640 | CCT CGG TCT CCC ACG GTC TCG |
| 5'NGSPas | 2592-2572 | GGG ATG TCG ACC CGA GGC TTT |
| GAPDH s | - | TGG ATA TTG TTG CCA TCA ATG ACC |
| GAPDH as | - | GAT GGC ATG GAC TGT GGT CAT G |
| | | |

| **b: Primers used for Genomic PCR and long PCR amplification** | | |
|---|---|---|
| **Primer** | **Nucleotide positions in TTV** | **SEQUENCE 5'- 3'** |
| primer A | 3211-3187 | CGA AAC GCG CAA GCG TTT CGG GTG G |
| primer B | 3293-3317 | GAA AGT GAG TGG GGC CAG ACT TCG C |
| tth25-1s | 126-156 | CCG CAG CGA GAA CGC CAC GGA GGG AGA TCC T |
| tth25-2as | 125-95 | ACG GGC GTG GAA AAC TCA GCC ATT CGG AAG T |
| tth25-7s | 128-156 | GCG GCG AGA ACG CCA CGG AGG GA |
| tth25-8as | 127-101 | GGA CGG GCG TGG AAA ACT CAG CCA TTC |
| jt34-1s | 223-247 | GGC CGG GCC ATG GGC AAG GCT CTT A |
| jt34-2as | 220-195 | TCC CGA GCC CGA ATT GCC CCT TGA CT |
| jt34-3s | 183-209 | TTT ACA CAC CGC AGT CAA GGG GCA ATT |
| jt34-4as | 182-153 | CTC ACC TCC GGC ACC CGC CCT CGG GTA CGT T |
| tth8-1s | 133-164 | AGA GGA GCC ACG GCA GGG GAT CCG AAC GTC CT |
| tth8-2as | 132-102 | CCG CGG ACG GGC AGC AAA AAC TCA GCC ATT C |

| | | |
|---|---|---|
| s* - forward as** - reverse | | |

Laboratory cross-contamination of these human samples with previously described tth-TT virus isolates (1) is highly unlikely as TT viruses were not included as positive controls in any of the PCR amplifications of the serum samples. The *in vitro* studies performed on two of our isolates, tth7 and tth8, were performed in separate locations.

The *in vitro* transfected tth8 DNA was amplified by long PCR with the primers A and B (Table 1) at 95°C for 45 sec followed by 5 cycles of 1 min at 95°C and 5 min at 72°C, 5 cycles of denaturation for 1 min at 95°C, annealing for 1 min at 70°C and elongation for 5 min at 72°C, 30 cycles of 1 min at 95°C, 1 min at 68°C and 5 min at 72°C and final elongation for 5 min at 72°C. Full-length tth8 genome was amplified as control for the long PCR reaction. Amplification of DNA from serum samples was initially performed using the primer combination NG133 and NG352 (resulting in an amplicon of 162 bp) followed by nested amplification with primers NG249 and NG351 (134 bp) (12), as well as amplification with the primers NG472 and NG352 (resulting in an amplicon of 91 bp) followed by nested NG473 and NG351 (71 bp) (7) (Table 1). The resulting amplicons were separated by gel electrophoresis and eluted from the gel with the Gel Extraction Spin Kit (Genomed, Löhne, Germany). Amplicons were each cloned into the vector pCR2.1 (TA Cloning Kit, Invitrogen, Carlsbad, CA, USA) prior to sequencing. At least 10 clones from each amplicon were sequenced. Serum samples harboring TT virus DNA were subsequently subjected to long PCR amplification as described above. Primers for this amplification (Table 2) were designed on the respective 70 bp sequences obtained after amplification with the primers NG473 and NG351. All obtained amplicons were eluted after gel electrophoresis, cloned and sequenced.

### (D) Southern blot analysis

*In vitro* replication of the viral DNA was monitored by transfecting L428 cells with the excised and linearized full length tth8 genome. These cells were harvested at day 1, 2, 3, and 7. Total cellular DNA was isolated and 10 µg digested with Dpn1 (Fermentas) and AflII (New England Biolabs, Frankfurt, Germany). DNA samples were subjected to gel electrophoresis after which the DNA was transferred onto a Hybond-N+ membrane (GE Healthcare). Hybridization, as well as labelling of the tth8 ORF1 for use as probe, was performed using the AlkPhos Direct labelling kit (GE Healthcare). The blot was exposed for 5 days using Kodak MS Biomax Film (Eastman Kodak).

### (E) RNA isolation and cDNA synthesis

Total RNA was isolated from transfected cells using the mirVana kit (Ambion, Austin, TX, USA) according to the manufacturer's protocol. All RNA samples were treated with DNaseI (New England Biolabs) to remove any residual DNA, followed by acid phenol (pH 4.8)/chloroform extraction and precipitation with ethanol. One µg of RNA was reverse transcribed at 42°C for 1 hour using 4 U OmniScript reverse transcriptase and 1 µM Oligo (dT)₁₂₋₁₈ primer according to the manufacturer's protocol (OmniScript Reverse Transcription Kit, Qiagen, Hilden, Germany). A second method for cDNA synthesis aimed at preventing secondary structure formation in RNA, was performed for 1 hour at 65°C using 15 U ThermoScript reverse transcriptase (Invitrogen) and 1 µM Oligo(dT)₂₂ primer. The efficiency of cDNA synthesis was equal in all preparations, as was evidenced using primers specific for the GAPDH gene. cDNA samples equivalent to 20 ng of total RNA were used as template for each PCR amplification which was performed in a final volume of 50 µl using specific tth8 primers (Table 1). Ten µl of the reaction mixture were analysed by gel electrophoresis after 30, 33 and 36 cycles of amplification. PCR amplification was terminated after 39 cycles in samples where no amplification was observed. All experiments were reproduced at least twice using different cDNA preparations.

The 5'- and 3'-ends of the cDNA encoding tth8 were amplified by rapid amplification (RACE) using the BD SMART RACE cDNA Amplification Kit (Clontech) according to the manufacturer's recommendations. The primers for 5'-RACE are listed in Tables 1 and 2. Briefly, 400 ng of DNase-treated RNA were reverse transcribed with BD PowerScript reverse transcriptase for 1.5 hours at 42°C. An aliquot of the reaction mixture was PCR amplified using UPM and GSP1 primers and the Advantage 2 PCR Kit reagents as recommended (Clontech). The specificity of amplification was increased by using the following conditions: 5 cycles of 30 sec at 94°C, annealing-extension for 3 min at 72°C, 5 cycles of 30 sec at 94°C (denaturation), 30 sec at 70°C (annealing), 3 min at 72°C (extension) and finally, 25 cycles of 30 sec at 94°C, 30 sec at 69°C and 3 min at 72°C. Amplicons were analysed by gel electrophoresis in 2% agarose gels, eluted and cloned into the vector pCR2.1 (Invitrogen). A total of 70 clones were randomly selected and sequenced.

### (F) Sequence analyses

All sequences were compared to sequences available in all databases by using the HUSAR software package (13, 14).

### Example 2

### In vitro replication of tth8

A cell culture system in which TT viruses will readily replicate, is not yet available. The release of TT virus particles into the supernatant of infected PHA-stimulated TT virus-negative PBMC cultures and infected Raji cell line has, however been described and viral load measured by PCR amplification of partial genome fragments (7). Here the replication of the full-length genome of tth8 already 48 hours after transfection into the L428 cell line is reported (Figure 1A). The replicating tth 8 genomic DNA increased up to day 7 after transfection, at which point almost no input DNA was present as measured by DpnI digestion. The complete tth8 genome was linearized at the SalI restriction site prior to transfection. Re-circularization of the genome occurred in the cell as evidenced by the AflII linearized replicative form (Figure 1b) and amplification of cDNA covering the region of the genome harboring the SalI restriction site (data not shown). Electronmicrographs demonstrated viral-like particles of the expected size of 30-40 nm (Figure 2).

### Example 3

### In vitro intragenomic rearrangement of tth7 and tth8

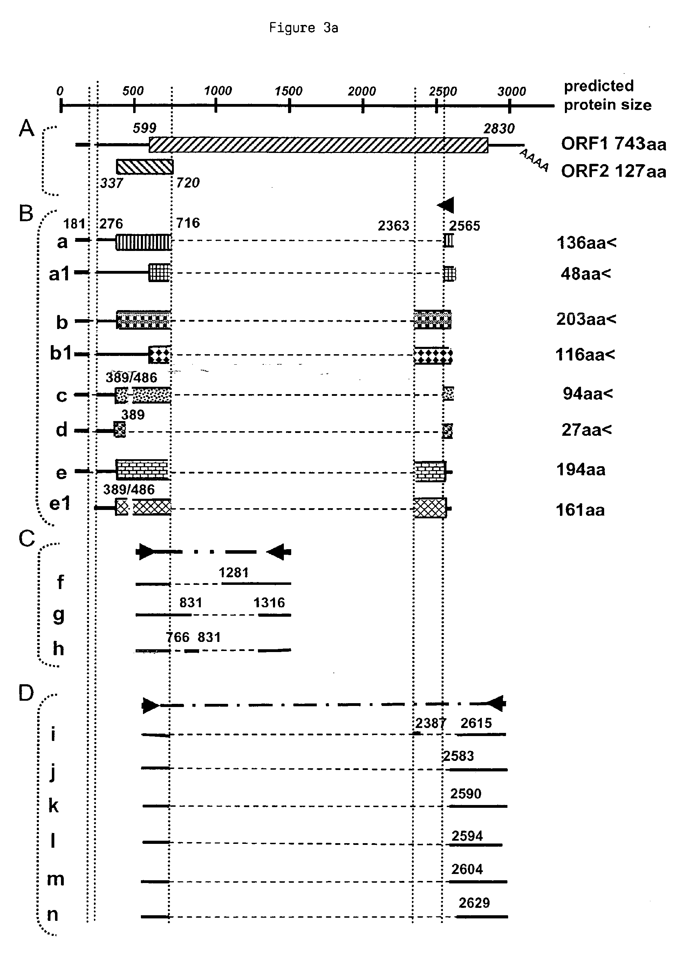

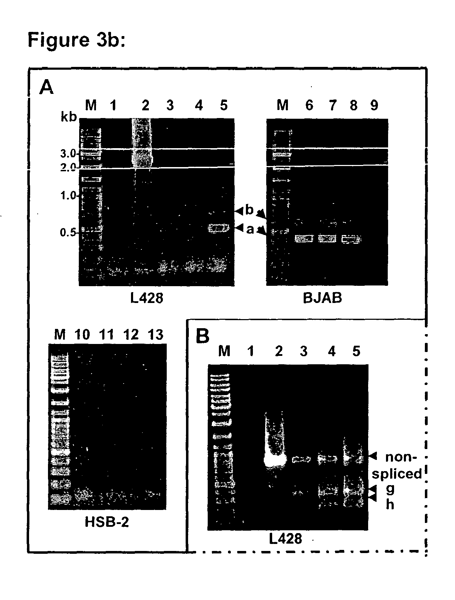

Additional off-sized amplicons were obtained after long PCR amplification for the almost full length genome (primers A and B, Table 1). Transfected cells harvested after 48 hours were pelleted and total DNA extracted. An example is presented in Figure 1c. The primers used for the long amplification of the tth8 genome will not amplify fragments of the Dpn1 digested input DNA. Sequence data revealed intragenomic rearrangement of the viral DNA which occurred mainly at the repetitive sequences in the tth8 genome. This probably indicates the formation of hairpin structures in the recombination event. Similar experiments were performed by transfecting only the tth7 ORF1. Here the off-sized bands again resulted from intragenomic rearrangement events occurring at nucleotide repeats which were mirrored in the modified amino acid sequence of ORF1 (Figure 3 and Table 2).

### Example 4

### In vitro transcription of tth8

*In vitro* transcription of full-length TT virus genomes has been reported (5,16), confirming the three mRNA species of 2.9 kb, 1.2 kb and 1.0 kb previously described in bone marrow cells (17). The group of tth-isolates closest related to tth8 varied only 1% in their sequence homology, but nevertheless harboured point mutations throughout the genome which resulted in fractionation of the ORF1 into 2 or more, shorter open reading frames. The alternative splicing events within the tth7 genome which were previously predicted (1), were recently confirmed for the TT virus genotype 6 (5). The tth8 genome was transfected into the L428 Hodgkin's lymphoma cell line and RACE analyses on the viral transcripts were performed. A total of 105 cDNA clones were sequenced and analysed. A series of primer combinations was used to amplify specific regions within each of the cDNA clones.

The tth mRNA species with the respective splice-donor and splice acceptor sites and their deviations from the consensus sequences (18) are listed in Table 2.

**Table 2**

| **tth7 and tth8 mRNA species with the respective splice-donor and - acceptor sites and their deviations from the consensus sequence** | | | | |
|---|---|---|---|---|
| **tth transcript** | **splice-donor site** | | **splice-acceptor site** | |
| | **nt** | **consensus** | **nt** | **consensus** |
| | **no** | A          A AGGT AGT C          G | **no** | CCCCCCCCCCCCCCC   A AG TTTTTTTTTTTTTTT   G |
| **a, a1**^{1,2} | 181 | G**AGGTGAGT** | 276 | **TTTTC**GAATAAGC**AGA** |
| | 716 | G**AAG**A**GTAA**GG | 2565 | **TT**G**T**A**TTCTCAGG** |
| **b, b1, e**^{1,2} | 181 | **GAGGTGAGT** | 276 | **TTTTCG**AA**T**AAG**CAGA** |
| | 716 | G**AAG**A**GTAA**GG | 2363 | **TC**A**TT**GAA**CAGA** |
| **c**^{1,2} | 181 | G**AGGTGAGT** | 276 | **TTTTC**GAA**T**AAG**CAGA** |
| | 389 | G**TGGTA**T**G**A | 486 | **CC**A**TCC**AA**CAGG** |
| | 716 | G**AAG**A**GTAAG**G | 2565 | **TT**G**T**A**TTCTCAGG** |
| **d**¹ | 181 | G**AGGTGAGT** | 276 | **TTTTC**GAA**T**AAG**CAGA** |
| | 389 | G**TGGTA**T**G**A | 2565 | **TT**G**T**A**TTCTCAGG** |
| **e1**^{1,2} | 181 | G**AGGTGAGT** | 276 | **TTTTC**GAA**T**AAG**CAGA** |
| | 389 | G**TGGTA**T**G**A | 486 | **CCATCC**AA**CAGG** |
| | 716 | G**AAG**A**GTAAG**G | 2363 | **TCATT**GAA**CAGA** |
| **f**² | 716 | **GAAG**A**GTAAG**G | 1281 | **CTCTTT**A**CAGA** |
| **g**² | 831 | ct**G**ca**GTA**A**T** | 1316 | ACctcaCCCTTTT |
| **h**² | 701 | ctAGACgac | 766 | A**ctT**A**CAga**c |
| | 831 | ct**G**ca**GTA**A**T** | 1316 | ACctcaCCCTTTT |
| **i**² | 716 | G**AAG**AGT**AAG**G | 2363 | **TC**A**TT**GAA**CAGA** |
| | 2387 | ctTCcaGCca | 2615 | AAGAAGGAA**AG**ctca |
| **j**² | 716 | **GAAG**A**GTAAG**G | 2583 | CGGGTCGAC |
| **k²** | 716 | G**aag**a**GTa**a**G**G | 2590 | ATCCCAAaaCAGaa |
| **I²** | 716 | G**aag**a**GTaag**G | 2594 | CCaaAACaag |
| **m²** | 716 | G**aag**a**GTaag**G | 2604 | CaagaAACCCaaga |
| **n²** | 716 | G**aag**a**GTaag**G | 2629 | ACTCCAaag |

| | | | | |
|---|---|---|---|---|
| ^{1, 2} - detected by RACE¹ and by RT-PCR² Bold and underlined - key nucleotides in concensus sequence Bold - nucleotides identitical with concensus Lower case - possible repetitive sequences | | | | |

Also included here are the junction sequences in the tth8 genome where apparent intragenomic rearrangement had taken place. These intragenomic rearrangement events were verified by repeating the cDNA synthesis with a reverse transcriptase which avoids the formation of RNA secondary structure artefacts. Figure 3a is a schematic presentation of the respective tth8 mRNA species. Transcripts a, a1, b, b1 and e correspond to the well characterized TT virus mRNAs (16, 5) and splicing at nucleotide positions for tth7 as predicted earlier (1). Transcripts c, d and e1 have not been described before. Transcript c resembles transcript a, except for additional splicing between nt 389 and nt 486 (Table 3) and transcript d is spliced between nt 389 and nt 2565. Transcript e1 also has the extra splicing site at nt 389 and nt 486. Both the transcripts e and e1 use the splice acceptor site at nt 2363 and have a stop codon at nt 2589. The splicing donor and acceptor signals at all 3 additional splicing sites were well conserved.

### Example 5

### In vitro transcription patterns of tth8 and tth7 in other hematopoietic cell lines

Hematopoietic cells act as the main reservoir for natural TT virus infections. It was investigated whether cell type would influence the *in vitro* transcription of tth7 in comparison with that of tth8. Transcriptions in the T-cell leukemia cell lines, Jurkat and HSB2 were analysed, as well as in the Burkitt lymphoma cell lines BJAB and Raji, and compared to the above described transcripts in the L428 Hodgkin lymphoma cell line. The human embryonic kidney cell line 293 was included based on the pathological changes reported in the kidneys of TT virus-ORF1 transgenic mice (19). Double-stranded DNA as replicative forms of the TT virus genome was demonstrated in liver cells in natural infections (20). Therefore, the HepG2 was included in the experiments. Each of these cell lines were transfected with the full-length tth7 and tth8 genomes. Total RNA was isolated 48 hours later and oligo(dT) cDNA synthesized. Transcription patterns were analysed by PCR amplification with a series of primers located within the ORF1 (Table 1). Transcripts covering almost the complete ORF1 (primers ORF1s and ORF1as) were present in all cell lines except in the HSB2 cell line (Figure 3b). Any other tth transcripts could not be detected in this cell line despite including positive controls for transfection and cDNA synthesis. The copy number of transcript b seemed to be higher in the BJAB and 293 cells than in the other cell lines. Transcription patterns did not differ between tth7 and tth8. Amplification with primers h7g43f and h7g43r within ORF1 (Table 1, 16) revealed an additional group of transcripts in the 293, Jurkat, L428 and BJAB cell lines (Table 2 and Figure 3). Transcript f resulted from splicing at nt 716 and nt 1281 where splicing consensus sequences are present. Two additional transcripts present only in the L428 cells, harbored junctions between nt 831 and nt 1316 in transcript g and between nt 701 and nt 766, as well as between nt 831 and nt 1316 in transcript h. As only one conserved splice donor site is present at nt 831, the other junctions may have resulted from intragenomic rearrangement. The transcripts i, j, k, 1, m and n, which were identified with primers ORF1s and ORF1as, had junctions between the splice donor site at nt 716 at the 5'end and varying nucleotides located between nt 2387 and nt 2629 at the 3'-end. Tandem repeats were present in this region of the genome (Table 2). These latter transcripts all contained open reading frames of which the predicted protein represented partial ORF1s varying in size. These results indicate a plurality of *in vitro* transcripts from one TT virus probably arising through intragenomic rearrangement, but opening the possibility for the expression of a variety of modified proteins.

### Example 6

### In vivo intragenomic rearrangement of TT virus genomes

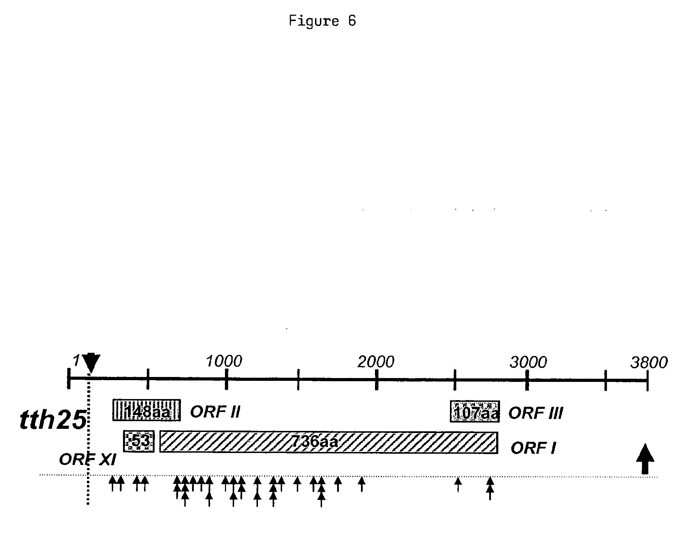

Data on the prevalence of TT virus infections in blood samples from lymphoma and leukemia patients are contradictory (8, 9, 10). Transplacental infection with TT virus has been described (21, 22, 23, 24). 60 serum samples were examined for the presence of TT virus DNA. The samples were taken from 30 pregnant women of whom the children developed leukemia within the first year of life, in comparison to 30 similar samples from a group control mothers. The highly conserved region of the TT virus control region was amplified in order to cover as broad a spectrum of TT virus types as possible (12). The 3'-half of this 170 bp conserved region (135 bp by nested amplification) is even more conserved (91 bp, nested amplification 71 bp) and amplification allows the detection of additional TT virus types (7). Both of these regions were amplified in all samples. All amplicons were cloned and sequenced. TT virus sequences were detected in 39 of 60 (65%) sera by amplifying the 170 bp region and 58 of 60 (97%) samples after amplification of the 71 bp. The high sequence homology between TT viruses in this 71 bp stretch does not permit the unequivocal identification of TT virus type. This sequence information was used and back to back primers (Table 1) for amplification of complete genomes from the serum samples were designed. DNA samples from 36 sera were first subjected to rolling circle amplification (TempliPhi) before amplification with the respective back to back primers. Different primers were designed differing in single nucleotides and/or length in order to optimize amplification conditions. The use of each of these combinations on each individual serum sample resulted in amplicons differing in size range. Amplicons equalling the size of complete genomes were obtained in a number of samples (Figure 4), as well as additional subgenomic amplicons. The number and size of the subgenomic amplicons varied between serum samples, although some selection was evident. The majority of the subgenomic amplicons was isolated and cloned. 296 sequences having the respective 5'- and 3'- primer sequences were analysed. The latter was taken as indication for a circular conformation of these isolates in the sera. The isolates ranged from 172 bp to full length genomes. All sequences were compared by BLAST analyses to TT virus sequences available in the databanks. The rearrangement of the subviral sequences did not allow for the clear-cut identification of novel TT virus types. Examples are depicted in Figure 5. All putative proteins coded for by ORFs present in the subviral DNA were analysed for similarities to known proteins (Domainsweep, 13) The genome organization of the closest related known TT virus type is shown and similarity of the putative protein from the ORFs to TT virus proteins indicated. The degree of rearrangement seems to vary between serum samples. Five serum samples harboured TT virus sequences closest related to TT virus tth25 (acc. no. aj620222, 16) (Figure 5a). Of these, thirteen subviral isolates from serum no. 74 range in size between 631 bp and 230 bp. The genome organization is largely retained, despite the decrease in size. ORF1 is located on the positive strand, although the putative protein from a larger ORF on the negative strand also shares similarity to the ORF1 protein. Similarly, putative proteins encoded by ORFs on the negative strand in 2 isolates from serum no. 52 shared similarity to the ORF3 protein. The 11 isolates from no. 52 range in size from 367 bp to 1361 bp. The 367 bp isolate shares closest sequence homology to tth25, although the single ORF (larger than 50 amino acids) present, does not show any similarity to TT virus proteins. The 539 bp isolate contained a shorter ORF2 (110 aa), whereas ORF1 is absent. ORFs in the 4 largest isolate (1287 bp to 1361 bp) with protein similarity to ORF1, vary between 52 aa and 73 aa in size. One of these isolates (1287 bp) had a larger ORF2 (161 amino acids). Serum no. 52 also harboured recombined isolates with closest homology to the TT mini-like virus tlmv-nlc030 (acc. no. ab038631, 54) (Figure 5b). ORF2 was absent from these subviral isolates. Other serum samples with subviral isolates closest related to tth25 were no. 49, no. 71 and no. 82. An interesting feature of the 8 isolates (1100 nt to 1462 nt) from serum no. 82 is the rearrangement of the ORF1 which is mostly represented by 2 ORFs combined with extra undefined ORFs in the ORF1 region of the sequence. The ORF2 remained intact in all isolates from no. 82, but was deleted in 3 of 7 isolates from no. 71 (5 isolates ranging from 965 bp to 1624 bp). Examples of corresponding nucleotide positions within the tth25 genome where break points in the rearranged sequences were located are depicted in Figure 6. Distinct nucleotide patterns could not be determined although the majority occurred in GC-rich areas of the sequence.

A second group of 4 serum samples had TT virus sequences closest related to the TT virus tchn-a (acc. no. af345526, 24) (Figure 5c). Sample no. 45 shared the same sequence of 737 bp with serum no. 46. Five additional recombined subviral isolates from serum no. 45 ranged from 375 bp to 1110 bp in length and 4 additional from serum no. 46 ranged from 855 bp to 1146 bp. The 5'-part of the genome organization was more or less retained in all 5 subviral isolates from no. 46, in contrast to only 4 of the 6 isolates from no. 45. The ORFs in the no. 45 isolates which had similarities to ORF2 and ORF1, were fragmented in combination with additional undefined ORF. Two subviral isolates from serum no. 53 were 172 bp and 737 bp in length. The 172 bp sequences formed part of an ORF of which the putative protein shared similarities to the ORF2 protein of TT virus. The second isolate harbored ORFs similar to the full length ORF2, as well as an overlapping ORFx1 (1).

Another TT virus type to which subviral isolates shared homology is TT virus saa-01 (acc. no. ab060597, 35) (Figure 5d). These isolates were cloned from 5 serum samples. One serum harboured 7 subviral isolates ranging from 387bp to 1509bp. Interesting is that the ORFs with similarity to ORF2 were either rearranged or absent. A similar phenomenon was present in the additional 2 isolates with similarity to TT virus saa-01, one 863bp in length (one serum sample) and the other 977bp in length (3 serum samples).

Two subviral isolates (561 bp and 565 bp) shared homology with TT virus tjn01 (57) (Figure 5e). The putative proteins from the only ORF present in both these clones showed similarity to ORF2 of TT virus. The one subviral clone (sle2460, 561bp) was isolated from 11 different serum samples and was the only clone in this collection isolated from such a large number of samples. The serum samples originated from 7 case mothers and 4 control mothers. This indicates a positive selection for this TT virus sequence suggesting that this TT virus isolate may contribute to the initiation of disease.

### Literature

**1.** Jelcic, I., A. Hotz-Wagenblatt, A. Hunziker, H. zur Hausen, and E-M. de Villiers. 2004. Isolation of multiple TT virus genotypes from spleen biopsy tissue from a Hodgkin's disease patient: genome reorganization and diversity in the hypervariable region. J. Virol. 78: 7498-7507.
**2.** Jones, M.S., A. Kapoor, V.V. Lukashov, P. Simmonds, F. Hecht, and E. Delwart. 2005. New DNA viruses identified in patients with acute viral infection syndrome. J. Virol. 79: 8230-8236.
**3.** Okamoto, H., M. Takahashi, T. Nishizawa, A. Tawara, K. Fukai, U. Muramatsu, Y. Naito, and A. Yoshikawa. 2002. Genomic characterization of TT viruses (TTVs) in pigs, cats and dogs and their relatedness with species-specific TTVs in primates and tupaias. J. Gen. Virol. 83: 1291-1297.
**4.** Takahashi, K., M. Hijikata, E.I. Samokhvalov, and S. Mishiro. 2000. Full or near full length nucleotide sequences of TT virus variants (types SANBAN and YONBAN) and the TT virus-like mini virus. Intervirol. 43: 119-123.
**5.** Qiu, J., L. Kakkola, F. Cheng, C. Ye, M. Soderlund-Venermo, K. Hedman, and D.J. Pintel. 2005. Human circovirus TT virus genotype 6 expresses six proteins following transfection of a full-length clone. J. Virol. 79: 6505-6510.
**6.** Miyata, H., H. Tsunoda, A. Kazi, A. Yamada, M.A. Khan, J. Murakami, T. Kamahora, K. Shiraki, and S. Hino. 1999. Identification of a novel GC-rich 113-nucleotide region to complete the circular, single-stranded DNA genome of TT virus, the first human circovirus. J. Virol. 73: 3582-3586.
**7.** Peng, Y.H., T. Nishizawa, T. Takahashi, T. Ishikawa, A. Yoshikawa, and H. Okamoto. 2002. Aanlysis of the entire genomes of thirteen TT virus variants classifiable into the fourth and fifth genetic groups, isolated from viremic infants. Arch. Virol. 147: 21-41.
**8.** Cacoub, P., E. Rosenthal, V. Gerolami, P. Hausfater, P. Ghillani, Y. Sterkers, V. Thibault, H. Khiri, J.C. Piette, and P. Halfon. 2003. Transfusion-associated TT virus co-infection in patients with hepatitis C virus is associated with type II mixed cryoglobulinemia but not with B-cell non-Hodgkin lymphoma. Clin. Microbiol. Infect. 9: 39-44.
**9.** Garbuglia, A.R., T. Iezzi, M.R. Capobianchi, P. Pignoloni, A. Pulsoni, J. Sourdis, E. Pescarmona, D. Vitolo, and F. Mandelli. 2003. Detection of TT virus in lymph node biopsies of B-cell lymphoma and Hodgkin's disease and its association with EBV infection. Int. J. Immunopathol. Pharmacol. 16: 109-118.
**10.** Shiramizu B., Q. Yu, N. Hu, R. Yanagihara, and V.R. Nerurkar. 2002. Investigation of TT virus in the etiology of pediatric acute lymphoblastic leukaemia. Pediatr. Hematol. Oncol. 19: 543-551.
**11.** Lehtinen M., P. Koskela, H.M. Ögmundsdottir, A. Bioigu, J. Dillner, M. Gudnadottir, T. Hakulinen, A. Kjartansdottir, M. Kvarnung, E. Pukkala, H. Tulinius, and T. Lehtinen. 2003. Maternal herpesvirus infections and risk of acute lymphoblastic leukaemia in the offspring. Am. J. Epidemiol. 158: 207-213.
**12.** Okamoto, H., M. Takahashi, N. Kato, M. Fukuda, A. Tawara, S. Fukuda, T. Tanaka, Y. Miyakawa, and M. Mayumi. 2000. Sequestration of TT virus of restricted genotypes in peripheral blood mononuclear cells. J. Virol. 74: 10236-10239.
13. Ernst P., K.H. Glatting, and S. Suhai. 2003. A task framework for the web interface W2H. Bioinformatics 19: 278-282.
**14.** Senger, M., T. Flores, K-H. Glatting, P. Ernst, A. Hotz-Wagenblatt, and S. Suhai. 1998. W2H: WWW interface to the GCG sequencwe analysis package. Bioinformatics 14: 452-457.
**15.** Desai, M., R. Pal, R. Deshmukh, and D. Banker. 2005. Replication of TT virus in hepatocyte and leukocyte cell lines. J. Med. Virol. 77: 136-143.
**16.** Kamahora, T., S. Hino, and H. Miyata. 2000. Three spliced mRNAs of TT virus transcribed from a plasmid containing the entire genome in COS1 cells. J. Virol. 74: 9980-9986.
**17.** Okamoto, H., A. Tawara, M. Takahashi, J. Kishimoto, T. Sai, and Y. Sugai. 2000. TT virus mRNAs detected bone marrow cells from an infected individual. Biochem. Biophys. Res. Commun. 279: 700-707.
**18.** Aebi, M., H. Hornig, R.A. Padgett, J. Reiser, and C. Weissmann. 1986. Sequence requirements for splicing of higher eukaryotic nuclear pre-mRNA. Cell 47: 555-565.
**19.** Yokoyama H., J. Yasuda, H. Okamoto, and Y. Iwakura. 2002. Pathological changes of renal epithelial cells in mice transgenic for the TT virus ORF1 gene. J. Gen. Virol. 83:141-150.
**20.** Okamoto, H., M. Ukita, T. Nishizawa, J. Kishimoto, Y. Hoshi, H. Mizuo, T. Tanaka, Y. Miyakawa, and M. Mayumi. 2000. Circular double-stranded forms of TT virus DNA in the liver. J. Virol. 74:5161-5167.
**21.** Goto, K., K. Sugiyama, T. Ando, F. Mizutani, K. Terabe, K. Tanaka, M. Nishhiyama, and Y. Wada. 2000. Detection rates of TT virus DNA in serum of umbilical cord blood, breast milk and saliva. Tohoku J. Exp. Med. 191: 203-207.
**22.** Matsubara H., K. Michitaka, N. Horiike, T. Kihana, M. Yano, T. Mori, and M. Onji. 2001. Existence of TT virus DNA and TTV-like mini virus DNA in infant cord blood: mother-to-neonatal transmission. Hepatol. Res. 21: 280-287.
**23.** Morrica, A., F: Maggi, M.L. Vatteroni, C. Fornai, M. Pistello, P. Ciccorossi, E. Grassi, A. Gennzzani, and M. Bendinelli. 2000. TT virus: evidence for transplacental transmission. J. Infect. Dis. 181: 803-804.
**24.** Saback, F.L., S.A. Gomes, V.S. de Paula, R.R. da Silva, L.L. Lewis-Ximenez, and C. Niel. 1999. Age-specific prevalence and transmission of TT virus. J. Med. Virol. 59: 318-322.

## Claims

1. A TTV polynucleic acid comprising:
(a) the nucleotide sequence depicted in Figure 7;
(b) a nucleotide sequence having at least 90% identity to the nucleotide sequence of (a), or
(c) a fragment of the nucleotide sequence of (a) or (b).

2. An oligonucleotide primer comprising part of the TTV polynucleic acid of claim 1, said primer being capable of acting as primer for specifically sequencing or specifically amplifying the nucleic acid of a certain TTV isolate containing a nucleotide sequence of claim 1.

3. An oligonucleotide probe comprising part of the TTV polynucleic acid of claim 1, said probe being capable of acting as a hybridization probe for specific detection of the nucleic acid of a certain TTV isolate containing a nucleotide sequence of claim 1.

4. A vector containing a TTV polynucleic acid of claim 1, a oligonucleotide primer of claim 2 or a oligonucleotide probe of claim 3.

5. A host cell transfected with the vector of claim 4.

6. A polypeptide having an amino acid sequence encoded by a TTV polynucleic acid of claim 1.

7. An antibody which specifically binds to the polypeptide of claim 6.

8. A diagnostic kit containing a TTV polynucleic acid of claim 1, an oligonucleotide primer of claim 2, an oligonucleotide probe of claim 3, a polypeptide of claim 6 and/or an antibody of claim 7.

9. Use of a TTV polynucleic acid of claim 1, an oligonucleotide primer of claim 2, an oligonucleotide probe of claim 3, a polypeptide of claim 6 and/or an antibody of claim 7 as an early marker for the future development of cancer or an autoimmune disease.

10. The use of claim 9, wherein the cancer is childhood leukaemia.

11. A vaccine comprising a polypeptide of claim 6.

12. Use of the polypeptide of claim 6 for the preparation of a vaccine for immunization of a mammal against TTV infection said TTV comprising a polynucleic acid of claim 1.
